# EUROPÄISCHE PATENTSCHRIFT

(11) **EP 1 427 693 B1**
(45) Veröffentlichungstag und Bekanntmachung des Hinweises auf die Patenterteilung: **01.03.2006**
(21) Anmeldenummer: 02776929.8
(22) Anmeldetag: 12.08.2002
(51) Int. Cl.: C07C 68/00, C08G 64/30

(54) **VERFAHREN ZUR HERSTELLUNG VON DIARYLCARBONATEN**
METHOD FOR PRODUCING DIARYLCARBONATES
PROCEDE DE PRODUCTION DE DIARYLCARBONATES

(30) Priorität: 24.08.2001 DE 10141622
(43) Veröffentlichungstag der Anmeldung: 16.06.2004
(73) Patentinhaber: Bayer MaterialScience AG, 51368 Leverkusen (DE)
(72) Erfinder: REISINGER, Claus-Peter, Wixom, MI 48393 (US); HANSEN, Sven-Michael, 51373 Leverkusen (DE); FISCHER, Peter, 50676 Köln (DE)
(86) Internationale Anmeldenummer: PCT/EP2002/008998
(87) Internationale Veröffentlichungsnummer: WO 2003/018526

(56) Entgegenhaltungen:
- EP-A- 0 583 938
- WO-A-02/057213
- US-A- 5 739 258
- US-A- 6 034 262

## Beschreibung

Die vorliegende Anmeldung betrifft ein Verfahren zur Herstellung von Diarylcarbonaten (DAC) dadurch gekennzeichnet, dass der als Edukt verwendete Hydroxyaromat zu bestimmten Zeitpunkten, abhängig von der Reaktionsführung, nachdosiert wird.

Die Herstellung von DAC durch oxidative Direktcarbonylierung von aromatischen Hydroxyverbindungen in Gegenwart von CO, O₂ und eines Edelmetall-Katalysators ist bekannt (siehe z.B. DE-OS 27 38 437, US-A 4,349,485, US-A 5,231,210, EP-A 667 336, EP-A 858 991, US-A 5,760,272). Als Edelmetall wird bevorzugt Palladium eingesetzt. Zusätzlich können ein Cokatalysator (z. B. Mangan- oder Kobaltsalze), eine Base, Bromidquellen, quaternäre Salze, verschiedene Chinone bzw. Hydrochinone und Trockenmittel eingesetzt werden. Dabei kann in einem Lösungsmittel gearbeitet werden.

Die bekannten Verfahren liefern jedoch keine für eine technische Umsetzung zufriedenstellenden Ausbeuten und erzeugen größere Mengen Nebenprodukte. Es ist daher wünschenswert ein Verfahren bereitzustellen, welches in bezug auf Ausbeute und Produktqualität optimiert ist.

Da es sich um Verfahren handelt, die ein Produkt in großtechnischem Maßstab bereitstellen sollen, bedeuten bereits kleine Optimierungen eine große Verbesserung.

Es wurde nun ein Verfahren gefunden, welches eine überraschende Steigerung der Selektivität der Reaktion, d.h. die Abnahme von Nebenprodukten bei gleichzeitig ansteigender Produktausbeute ermöglicht.

Gegenstand der Erfindung ist somit ein Verfahren zur Herstellung eines aromatischen Carbonats der Formel

(H-O)ₖ₋₁-R[-O-CO-O-R]ₙ-(O-H)ₖ₋₁ (I),

wobei k für ganze Zahlen von 1 bis 2, bevorzugt 1, und n für ganze Zahlen von 1 bis 30, bevorzugt 1 bis 10, besonders bevorzugt 1 und R für einen aromatischen Rest stehen,
bei dem eine aromatischen Hydroxyverbindung der Formel

R-(O-H)ₖ (II),

worin R und k wie oben definiert sind,
CO und O₂, sowie optional ein Lösungsmittel und verschiedene Katalysatorkomponenten zur Reaktion gebracht werden, dadurch gekennzeichnet, dass R-(O-H)ₖ mindestens einmal während der Reaktionszeit in die Reaktionsmischung nachdosiert wird.

Bei den erfindungsgemäß umsetzbaren aromatischen Hydroxyverbindungen R-(O-H)ₖ handelt es sich beispielsweise um Monohydroxyverbindungen (k=1, n=1) wie Phenol, o-, m- oder p-Kresol, o-, m- oder p-Chlorphenol, o-, m- oder p-Ethyphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol oder Dihydroxyverbindungen (k=2, 1 ≤ n ≤ 30, bevorzugt 1 ≤ n ≤ 10, besonders bevorzugt 1 ≤ n ≤ 5) wie Resorcin und Hydrochinon oder Bisphenole wie 2,2-Bis-(4-hydroxyphenyl)-propan (Bisphenol A), 2,2- Bis-(3,5-dimethyl-4-hydroxyphenyl)-propan, 1,1-Bis-(4-hydroxyphenyl)-cyclohexan, 1,1-Bis-(4-hydroxyphenyl)-3,3,5-trimethylcyclohexan oder 6,6'-Dihydroxy-3,3,3',3'-tetramethyl-1,1'-spiro(bis)-indan, 2,4'-Hydroxybiphenyl oder 4,4'-Hydroxybiphenyl. R-(O-H)ₖ kann verschiedene Substituenten am aromatischen Kern R besitzen. Allgemein handelt es sich im Falle einer Substitution der aromatischen Hydroxyverbindung um 1 bis 3 Substituenten in der Bedeutung von C₁-C₁₈-Alkyl, C₆-C₁₈-Aryl, C₇-C₁₈-Aralkyl, C₁-C₁₈-Alkoxy, Fluor, Chlor oder Brom, wobei die Substituenten, im Falle der Kohlenwasserstoffe, ihrerseits wieder ein- oder mehrfach mit den genannten Gruppen substituiert sein können.

Es können Gemische verschiedener R-(O-H)ₖ verwendet werden, bevorzugt ist jedoch die Verwendung einer einzigen Verbindung R-(O-H)ₖ. Bevorzugt wird Bisphenol A eingesetzt. Bevorzugt werden weiterhin die oben genannten Monohydroxyverbindungen eingesetzt, besonders bevorzugt Phenol, o-, m-, und p-Kresol, ganz besonders bevorzugt Phenol.

Das Reaktionssystem der oxidativen Direktcarbonylierung enthält typischerweise mehrere der folgenden Katalysatorkomponenten:
a) ein Metallsalz der Gruppe VIIIB,
b) mindestens ein zweites Metallsalz,
c) eine Bromidquelle
d) eine Base
e) organische Cokatalysatoren

Die für das erfindungsgemäße Verfahren geeigneten Platinmetall-Katalysatoren a) bestehen aus mindestens einem Edelmetall der Gruppe VIII, vorzugsweise Palladium. Es kann bei dem erfindungsgemäßen Verfahren in verschiedener Form zugegeben werden. Palladium kann in metallischer Form z.B. als Palladiumschwarz oder auf einem Träger wie Pd/C, Pd/Al₂O₃, Pd/SiO₂ oder bevorzugt in Form von Palladium-Verbindungen der Oxidationsstufen 0 und +2, wie beispielsweise Palladium(II)-acetylacetonat, -halogenide, -carboxylate von C₂-C₁₈-Carbonsäuren, -dicarboxylate wie Oxalat, -nitrat, -sulfat -oxide oder Palladiumkomplexe, die beispielsweise Kohlenmonoxid, Olefine, Amine, Nitrile, Phosphorverbindungen und Halogenide enthalten können, eingesetzt werden. Besonders bevorzugt sind Palladiumbromid und Palladiumacetylacetonat.

Die Menge an Platinmetall-Katalysator ist im erfindungsgemäßen Verfahren nicht beschränkt. Bevorzugt wird so viel Katalysator zugesetzt, dass die Konzentration des Metalls im Reaktionsansatz 1 bis 3000 ppm beträgt, besonders bevorzugt sind Konzentrationen von 5 bis 500 ppm.

Als zweites als Cokatalysator wirkendes Metallsalz b) für das erfindungsgemäße Verfahren wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew), ggfs. auch Mischungen davon, verwendet, wobei das Metall in verschiedenen Oxidationsstufen eingesetzt werden kann. (s. z. B. US-A 5,142,086, US-A 5,231,210, US-A 5,284,964, EP-A 350 697, EP-A 350 700, US-A 5,336,803) Bevorzugt werden Pb, Ti, Mn, Cu, Co, V, Zn, Ce und Mo eingesetzt. Ohne das erfindungsgemäße Verfahren einzuschränken, seien Blei (ll), Man gan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₁₈-Carbonsäuren, Diketonate oder Nitrate sowie als Komplexverbindungen eingesetzt werden, die beispielsweise Kohlenmonoxid, Olefine, aromatische und aliphatische Mono- oder Polyamine, Phosphorverbindungen, Pyridine, Bipyridine, Terpyridine, Chinoline, Isochinoline, Kryptanden, Schiffbasen und Halogenide enthalten können. Besonders bevorzugt werden Mn, Cu, Mo, Pb und Ce eingesetzt Ganz besonders bevorzugt werden Manganverbindungen im erfindungsgemäßen Verfahren verwendet, besonders bevorzugt Mangan(II)- und Mangan(III)komplexe, ganz besonders bevorzugt Mangan(II)acetylacetonat bzw. Mangan(III)-acetylacetonat, sowie Mangan(II)bromid.

Der Cokatalysator, der auch in-situ gebildet werden kann, wird in einer solchen Menge zugesetzt, dass seine Konzentration im Bereich von 0,0001 bis 20 Gew.-% des Reaktionsgemisches liegt, bevorzugt ist der Konzentrationsbereich von 0,001 bis 5 Gew.-%, besonders bevorzugt 0,005 bis 2 Gew.-%.

Bei den im Rahmen der vorliegenden Erfindung eingesetzten Bromidverbindungen c) handelt es sich beispielsweise um die Alkalibromide oder Erdalkalibromide, bevorzugt aber um die Bromidsalze von organischen Kationen. Bei den organischen Kationen kann es sich beispielsweise um mit organischen Resten substituierte Ammonium-, Guanidinium-, Phosphonium- oder Sulfoniumsalze, ggfs. auch Mischungen davon, handeln. Geeignet für den Einsatz in das erfindungsgemäße Verfahren sind Ammonium-, Guanidinium-, Phosphonium- und Sulfoniumsalze, die als organische Reste C₆- bis C₁₈-Aryl-, C₇- bis C₁₈-Arlkyl- und/oder C₁- bis C₂₀-Alkyl-Reste enthalten. Bevorzugt werden in das erfindungsgemäße Verfahren Ammoniumsalze eingesetzt, die als organische Reste C₆- bis C₁₈-Aryl-, C₇- bis C₁₈-Aralkyl- und/oder C₁₋bis C₂₀-Alkyl-Reste tragen, besonders bevorzugt sind Tetrabutylammoniumbromid, Tetraphenylphosphoniumbromid und Tetrabutylphosphoniumbromid. Die Menge eines solchen quaternären Salzes kann beispielsweise 0,1 bis 20 Gew.-%, bezogen auf das Gewicht des Reaktionsgemisches, betragen. Vorzugsweise beträgt diese Menge 0,5 -15 Gew.-%, besonders bevorzugt 1- 5 Gew.-%.

Für das erfindungsgemäße Verfahren einsetzbare Basen sind Alkalihydroxide, Alkalisalze bzw. quaternäre Salze von schwachen Säuren wie Alkali-teri.-butylate oder Alkalisalze bzw. quaternäre Salze von aromatischen Hydroxyverbindungen der Formel (II), in der R die oben angegebene Bedeutung hat. Ganz besonders bevorzugt wird ein Alkalisalz bzw. quaternäres Salz der aromatischen Hydroxyverbindung der Formel (II) verwendet, die auch zum organischen Carbonat umgesetzt werden soll, beispielsweise Tetrabutylammonium- oder Kaliumphenolat.

Die Alkalisalze können Lithium-, Natrium-, Kalium-, Rubidium- oder Cäsiumsalze sein. Bevorzugt werden Lithium-, Natrium-, und Kaliumsalze, besonders bevorzugt Kaliumsalze eingesetzt, ganz besonders bevorzugt Kaliumphenolat.

Die quaternären Salze können Ammonium-, Phosphonium-, Pyridinium-, Sulfonium oder Guanidiniumsalze sein, die als organische Reste C₆- bis C₁₈-Aryl-, C₇- bis C₁₈₋Aralkyl- und/oder C₁- bis C₂₀-Alkyl-Reste besitzen. Die Reste können alle gleich oder verschieden sein, ggfs. können auch Mischungen mehrerer Quartärsalze eingesetzt werden. Bevorzugt wird dabei ggfs. das selbe Kation eingesetzt, das auch als Bromid für Komponente c) verwendet wird. Weiterhin bevorzugt werden Tetraphenylphosphonium, Tetrabutylammonium-, Tetrabutylphosphonium, besonders bevorzugt wird Tetrabutylammonium.

Alternativ können auch Trialkylaminbasen wie Tributylamin, Diisopropylethylamin, DBU, DBN verwendet werden.

Die Base d) wird in einer von der Stöchiometrie unabhängigen Menge zugesetzt. Das Verhältnis von Platinmetall, z.B. Palladium, zu Base wird vorzugsweise so gewählt, dass pro mol Platinmetall, z.B. Palladium, 0,1 bis 5000, bevorzugt 1 bis 1000, besonders bevorzugt 10 bis 300 Äquivalente Base eingesetzt werden.

Als organische Cokatalysatoren e) können z. B. erschiedene Terpyridine, Phenanthroline, Chinoline und Isochinoline, Semichinone, Hydrochinone und Chinone verwendet werden oder es werden die in EP1024132-A1 genannten organischen Cokatalysatoren eingesetzt.

Konkrete Beispiele sind 2,2':6',2"-Terpyridin, 4'-Methyltbio-2,2':6'2'-Teipyridin, 2,2':6',2"-Terpyridin-N-Oxid, 1,10-Phenanthrolin, 2,4,7,8-Tetramethyl-1,10-Phenanthrolin, 4,7-Diphenyl-1,10-Phenanthrolin, Catechol, 1,2-Chinon, Hydrochinon, p-Benzochinon Anthrachinon, 9,10-Dihydroxyanthracen und Phenanthrenchinon.

Der Hydroxyaromat R-(O-H)ₖ wird erfindungsgemäß portionsweise ein- oder mehrmals nachdosiert. Bevorzugt wird dabei mit einer innerhalb der ersten Stunde Reaktionszeit eingestellten Startkonzentration von etwa 10 bis 80 Gew.-% der Gesamtmenge begonnen und die Restmenge in einem oder mehreren Intervallen zugegeben. Besonders bevorzugt beträgt die Startkonzentration etwa 30 bis 70 Gew.-%. Bevorzugt wird die Zugabe in einer bis etwa zehn Portionen. Die Zeitintervalle werden bevorzugt etwa äquidistant gewählt. Bevorzugt wird eine Zugabe der letzten Portion, zu einem Zeitpunkt, der vor etwa 90 %, besonders bevorzugt vor etwa 80 % der gesamten Reaktionszeit, bzw. mittleren Verweilzeit liegt.

Die nachdosierte Menge R-(O-H)ₖ kann auch kontinuierlich eingeleitet werden, beispielsweise durch langsames Zupumpen einer Lösung. Bevorzugt wird dabei mit einer innerhalb der ersten Stunde Reaktionszeit eingestellten Startkonzentration von etwa 10 bis 80 Gew.%, besonders bevorzugt 30 bis 70 Gew.% der Gesamtmenge begonnen und die Restmenge anschließend kontinuierlich zudosiert. Dabei können verschiedene Zeit-Konzentrationsdosierprofile eingestellt werden, z. B. kann am Anfang eine größere Menge pro Zeiteinheit zudosiert werden als zum Ende der Reaktionszeit hin oder umgekehrt. Bevorzugt wird eine zeitlineare Zugabe der R-(O-H)ₖ₋Menge, bei der die zugegebene Menge pro Zeitintervall in etwa konstant bleibt Die Zugabe kann zu beliebigen Zeitpunkten während der Reaktionsdauer, bzw. der mittleren Verweilzeit begonnen und abgebrochen werden.

Bevorzugt wird eine Beendigung der Dosierung zu einem Zeitpunkt, der vor etwa 90 % besonders bevorzugt vor etwa 80 % der gesamten Reaktionszeit, bzw. mittleren Verweilzeit liegt.

Die Zudosierung von R-(O-H)ₖ kann als Feststoff oder als Flüssigkeit bzw. Lösung erfolgen. Ggfs. kann R-(O-H)ₖ über beheizte Zuleitungen zudosiert werden. Bevorzugt wird jedoch die Zugabe als Lösung in einem inerten Lösemittel.

Das erfindungsgemäße Verfahren kann dabei sowohl in kontinuierlicher wie auch diskontinuierlicher Weise geführt werden.

Bei einer diskontinuierlichen Durchführung wird R-(O-H)ₖ zu einem Zeitpunkt nach Beginn der Reaktion im Reaktor zudosiert.

Bei einer kontinuierlichen Durchführung kann R-(O-H)ₖ beispielsweise in einer Kaskade von m Reaktionsapparaten während der Reaktion in mindestens einen der 2. bis m.ten Reaktoren zudosiert werden.

Das erfindungsgemäße Verfahren zur Carbonatbildung wird bei einer Reaktionstemperatur von 30 bis 200°C, bevorzugt 50 bis 150°C, besonders bevorzugt 60 bis 130°C, und bei einem Reaktionsdruck von 1 bis 200 bar, bevorzugt 1 bis 50 bar, besonders bevorzugt 1 bis 10 bar durchgeführt.

Als inertes organisches Lösungsmittel können Kohlenwasserstoffe, halogenierte Kohlenwasserstoffe und aromatische Lösemittel wie Chlorbenzol, Dichlorbenzol, Fluorbenzol, Benzol, Toluol, Anisol, Cyclohexan, Petrolether, Methylenchlorid oder 1,2-Dichlorethan, dipolar aprotische Lösungsmittel wie Dimethylacetamid, Acetonitril, N-Methylpyrrolidinon, Ether wie Dioxan, Tetrahydrofuran, t-Butylmethylether und veretherte Glykole, ggfs. auch Mischungen verschiedener Lösungsmittel verwendet werden. Besonders bevorzugt wird Chlorbenzol eingesetzt. Das inerte Lösemittel kann mit einem Anteil von 1 bis 99 %, bevorzugt 20 bis 98 %, besonders bevorzugt 40 bis 98 %, in der Reaktionsmischung enthalten sein.

Die Zusammensetzung der Reaktionsgase Kohlenmonoxid und Sauerstoff kann in weiten Konzentrationsgrenzen variiert werden, es wird jedoch zweckmäßigerweise ein CO:O₂ Molverhältnis (normiert auf CO) von 1:0,001 bis 1:1, bevorzugt 1:0,01 bis 1:0,5 und besonders bevorzugt von 1:0,02 bis 1:0,3 eingestellt. Der Sauerstoff partialdruck ist bei diesen Molvechältnissen groß genug, um hohe Raum-Zeit-Ausbeuten erreichen zu können.

Alle Ausgangsverbindungen können mit Verumreinigungen aus ihrer Herstellung und Lagerung kontaminiert sein, jedoch ist es im Sinne der Reinheit des Endproduktes wünschenswert, mit möglichst sauberen Chemikalien zu arbeiten. Auch die Reaktionsgase unterliegen keinen besonderen Reinheitsanforderungen. So kann Synthesegas als CO-Quelle und Luft als O₂-Träger dienen, es ist jedoch darauf zu achten, dass keine Katalysatorgifte wie z.B. Schwefel oder dessen Verbindungen eingetragen werden. Die Gase können mit einem oder mehreren anderen Gasen wie Stickstoff Argon, Kohlendioxid oder Wasserstoff verdünnt sein. In einer bevorzugten Ausführungsform des erfindungsgemäßen Verfahrens werden reines CO und reiner Sauerstoff verwendet.

In einer weiteren Ausführungsform werden statt des homogenen Katalysatorsystems heterogene Katalysatoren, bei denen das Platinmetall oder das Platinmetall und/oder der Cokatalysator auf einem heterogenen Träger aufgebracht sind, als Pulver oder Formkörper eingesetzt. Die übrigen Komponenten des Katalysatorsystems, wie die Base, die quaternäre Verbindung und gegebenenfalls der Cokatalysator, sind weiterhin in der Reaktionslösung homogen gelöst. Der Anteil des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der oben genannten Art eingesetzt.

Der Mengenanteil des Cokatalysators am Gesamtgewicht des heterogenen Katalysators beträgt 0,01 bis 15 Gew.%, bevorzugt 0,05 bis 10 Gew.-% gerechnet als Metall.

Als Katalysatorträger eignen sich ein oder mehrere Metalloxide aus der Gruppe von V, Mn, Ti, Cu, Zr, La, der Seltenerdmetalle (Atomnummem 58-71), sowohl im Sinne chemisch einheitlicher Reinsubstanzen als auch im Gemisch sowie Eisen- und Kobaltoxide, Nckel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Ruhrgefäßen oder Blasensäulen werden Mengen von 0,001 bis 50 Gew.-%, bevorzugt von 0,01 bis 20 Gew.-%, besonders bevorzugt von 0,1 bis 10 Gew.-% Trägerkatalysator-Pulver bezogen auf die eingesetzte Menge an aromatischer Hydroxyverbindung, verwendet.

In bevorzugten Ausführungsformen wird der heterogene Trägerkatalysator ortsfest in Rührbehältern, einer Blasensäule, einem Rieselphasenreaktor oder Kaskaden dieser Reaktoren eingesetzt Eine Abtrennung des Trägerkatalysators entfällt dann völlig.

Als Reaktoren für das erfindungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß zu verwenden Rührbehälter mit dafür geeigneten Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können im erfindungsgemäßen Verfahren folgende Typen eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strablschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 51 (1979) Nr. 3, S. 208-216; W.-D. Deckwer, Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer wie im Falle eines Rührkessels oder durch andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet. Im Falle der Verwendung pulverförmigen Trägerkatalysatoren können diese aus der Reaktionsmischung z.B. durch Filtration, Sedimentation oder Zentrifugieren abgetrennt werden.

Bevorzugt, besonders bevorzugt oder ganz besonders bevorzugt sind Ausführungsformen, welche von den unter bevorzugt, besonders bevorzugt oder ganz besonders bevorzugt genannten Parametern, Verbindungen, Definitionen und Erläuterungen Gebrauch machen.

Die oben aufgeführten allgemeinen oder in Vorzugsbereichen aufgeführten Definitionen, Parameter, Verbindungen und Erläuterungen können jedoch auch untereinander, also zwischen den jeweiligen Bereichen und Vorzugsbereichen beliebig kombiniert werden.

In diskontinuierlichen Versuchen verwendete Trägerkatalysatoren können bei gleichen Einsatzstoffen ggfs. ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch ggfs. regeneriert werden.

### Beispiele

Die Reaktionskomponenten werden durch Gaschromatographie untersucht, wobei die Massen der Komponenten mittels eines internen Standards bestimmt werden können. Die Selektivität wird berechnet, indem die Menge des in der Reaktionsmischung enthaltenen Restphenols und des zu DPC umgesetzten Phenols zusammenaddiert und durch die eingesetzte Gesamtphenolmenge dividiert werden. Das dabei nicht erfasste Phenol wurde zu Nebenprodukten umgesetzt.

### Beispiel 1

In einen Autoklaven werden 0,03 mmol Palladium(II)bromid, 7,5 mmol Tetrabutylammoniumbromid, 0,7 mmol Mangantrisacetylacetonat und 16 g Phenol in 50 ml Chlorbenzol vorgelegt und unter Durchleiten von Kohlenmonoxid auf 80°C erwärmt. Anschließend werden 7,5 mmol Tetrabutylammoniumphenolat in 30 ml Chlorbenzol zugegeben. Bei 90°C/3 bar wird eine Gasmischung aus Kohlenmonoxid und Sauerstoff (97:3 Vol.%), durchgeleitet. Nach 15 min. werden innerhalb von 90 min zusätzliche 8,2 g Phenol in 12,3 g Chlorbenzol, kontinuierlich und gleichmäßig zugepumpt. Nach 2 h wird die Reaktion abgebrochen und das Reaktionsgemisch mittels GC untersucht Die Ergebnisse sind in Tab. 1 zusammengefasst.

### Vergleichsbeisipiel 1

In einen Autoklaven werden 0,03 mmol Palladium(II)bromid, 7,5 mmol Tetrabutylammoniumbromid, 0,7 mmol Mangantrisacetylacetonat und 24,2 g Phenol in 50 ml Chlorbenzol vorgelegt und unter Durchleiten von Kohlenmonoxid auf 80°C erwärmt. Anschließend werden 7,5 mmol Tetrabutylammoniumphenolat in 30 ml Chlorbenzol zugegeben. Bei 90°C/3 bar wird eine Gasmischung aus Kohlenmonoxid und Sauerstoff (97:3 Vol.%), durchgeleitet Nach 2 h wird die Reaktion abgebrochen und das Reaktionsgemisch mittels GC untersucht. Die Ergebnisse sind in Tab. 1 zusammengefasst.

**Tabelle 1:**

| | Bsp. 1 | Vgl. 1 |
|---|---|---|
| Umsatz Phenol [%] | 36,9 | 35,9 |
| Selektivität [%] | 87,1 % | 83,8 % |
| Raum-Zeit-Ausbeute [g/l/h] | 35,1 | 33,5 |
| Turn-Over-Number Pd | 1216 | 1022 |

## Patentansprüche

1. Ein Verfahren zur Herstellung eines aromatischen Carbonats der Formel
(H-O)ₖ₋₁-R[-O-CO-O-R]ₙ-(O-H)ₖ₋₁
wobei k für ganze Zahlen von 1 bis 2 und n für ganze Zahlen von 1 bis 30 und R für einen aromatischen Rest stehen,
indem eine aromatischen Hydroxyverbindung der Formel R-(O-H)ₖ, Kohlenmonoxid und Sauerstoff sowie optional ein Lösungsmittel und verschiedene Katalysatorkomponenten zur Reaktion gebracht werden, und R-(O-H)ₖ mindestens einmal während der Reaktionszeit in die Reaktionsmischung nachdosiert wird, **dadurch gekennzeichnet, dass** die innerhalb der ersten Stunde der Reaktionszeit dosierte Menge R-(O-H)ₖ etwa 10 bis 80 Gew.-% der zudosierten Gesamtmenge beträgt und die Dosierung von R-(O-H)ₖ zu einem Zeitpunkt, der vor etwa 90 % der gesamten Reaktionszeit, bzw. mittleren Verweilszeit liegt, beendet wird.

2. Verfahren nach Anspruch 1, bei dem es sich bei (H-O)ₖ₋₁-R[-O-CO-O-R]ₙ₋(O-H)ₖ₋₁ um Diphenylcarbonat und bei R-(O-H)ₖ um Phenol handelt.

3. Verfahren nach Anspruch 1, bei dem es sich um die Herstellung eines Oligo- oder Polycarbonats aus Bisphenol A handelt.

4. Verfahren nach Anspruch 1, bei dem die Nachdosierung portionsweise in ein bis zehn Portionen erfolgt.

5. Verfahren nach Anspruch 1, bei dem die Nachdosierung kontinuierlich erfolgt.

6. Verfahren nach Anspruch 5, **dadurch gekennzeichnet, dass** die Herstellung des aromatischen Carbonats in einer Kaskade von m Reaktionsapparaten erfolgt, wobei R-(O-H)ₖ während der Reaktion in mindestens einen der 2. bis m.ten Reaktoren zudosiert wird.

## Revendications

1. Un procédé de production d'un carbonate aromatique de la formule
(H-O)ₖ₋₁-R[-O-CO-O-R]ₙ-(O-H)ₖ₋₁
dans laquelle k représente des nombres entiers de 1 à 2, n correspond à des nombres entiers de 1 à 30 et R équivaut à un radical aromatique,
dans lequel on fait réagir un composé hydroxylé aromatique de la formule R-(O-H)ₖ, du monoxyde de carbone et de l'oxygène ainsi que, facultativement, un solvant et différents composants catalyseurs, et R-(O-H)ₖ, est postdosé au moins une fois dans le mélange réactionnel pendant le temps de réaction, **caractérisé en ce que** la proportion dosée de R-(O-H)ₖ au cours de la première heure du temps de réaction atteint environ 10 à 80 % en poids de la proportion totale dosée et que l'adjonction dosée du R-(O-H)ₖ est terminée à un moment se situant avant environ 90 % du temps de réaction total ou d'un temps de séjour moyen.

2. Procédé selon la revendication 1, dans lequel le (H-O)ₖ₋₁₋R[-O-CO-O-R]ₙ-(O-H)ₖ₋₁ est du diphénylcarbonate et le R-(O-H)ₖ est du phénol.

3. Procédé selon la revendication 1, dans lequel il s'agit de la production d'un oligo- ou d'un polycarbonate de bisphénol A.

4. Procédé selon la revendication 1, dans lequel le postdosage a lieu par portions (de façon discontinue) en une à dix portions.

5. Procédé selon la revendication 1, dans lequel le postdosage a lieu de façon continue.

6. Procédé selon la revendication 5, **caractérisé en ce que** la production du carbonate aromatique a lieu dans une cascade de m appareils de réaction, le R-(O-H)ₖ étant ajouté par dosage au cours de la réaction dans au moins un des 2^{ème} à m^{ième} réacteurs.

## Claims

1. A process for the preparation of an aromatic carbonate of the formula
(H-O)ₖ₋₁-R[-O-CO-O-R]ₙ-(O-H)ₖ₋₁
wherein k represents integers of 1 to 2 and n represents integers from 1 to 30 and R represents an aromatic radical,
in which an aromatic hydroxy compound of the formula R-(O-H)ₖ, carbon monoxide and oxygen, and optionally a solvent and various catalyst components are reacted, and R-(O-H)ₖ is subsequently metered into the reaction mixture at least once during the reaction time, **characterized in that** the amount of R-(O-H)ₖ metered in within the first hour of the reaction time is about 10 to 80 wt.% of the total amount metered in and the metering of R-(O-H)ₖ is ended at a point in time which is before about 90% of the total reaction time or average residence time.

2. Process according to claim 1, in which (H-O)ₖ₋₁-R[-O-CO-O-R]ₙ-(O-H)ₖ₋₁ is diphenyl carbonate and R-(O-H)ₖ is phenol.

3. Process according to claim 1, for the preparation of an oligo- or polycarbonate from bisphenol A.

4. Process according to claim 1, in which the subsequent metering takes place in portions in one to ten portions.

5. Process according to claim 1, in which the subsequent metering takes place continuously.

6. Process according to claim 5, **characterized in that** the preparation of the aromatic carbonate takes place in a cascade of m reaction apparatuses, wherein R-(O-H)ₖ is metered into at least one of the 2nd to mth reactors during the reaction.
